# EUROPEAN PATENT SPECIFICATION

(11) **EP 1 520 010 B1**
(45) Date of publication and mention of the grant of the patent: **07.11.2007**
(21) Application number: 03723800.3
(22) Date of filing: 25.03.2003
(51) Int. Cl.: C12N 5/10, C12N 15/12, G01N 33/53, G01N 33/566

(54) **SYSTEMS AND METHODS FOR DETECTION OF NUCLEAR RECEPTOR FUNCTION USING REPORTER ENZYME MUTANT COMPLEMENTATION**
SYSTEME UND VERFAHREN ZUM NACHWEIS DER FUNKTION NUKLEÄRER REZEPTOREN UNTER VERWENDUNG VON REPORTERENZYMMUTANTEN KOMPLEMENTIERUNGEN
SYSTEMES ET PROCEDES DE DETECTION DE LA FONCTION DE RECEPTEURS NUCLEAIRES PAR COMPLEMENTATION DE MUTANTS ENZYMATIQUES RAPPORTEURS

(30) Priority: 25.03.2002 US 366524 P
(43) Date of publication of application: 06.04.2005
(73) Proprietor: Applera Corporation, Bedford, MA 01730 (US)
(72) Inventor: PALMER, Michelle, A., J., Arlington, MA 02174 (US)
(74) Representative: Schnappauf, Georg
(86) International application number: PCT/US2003/008767
(87) International publication number: WO 2003/090515

(56) References cited:
- US-B1- 6 270 964
- US-B1- 6 294 330
- US-B1- 6 342 345
- ROSSI F ET AL: "MONITORING PROTEIN-PROTEIN INTERACTIONS IN INTACT EUKARYOTIC CELLS BY BETA-GALACTOSIDASE COMPLEMENTATION" PROCEEDINGS OF THE NATIONAL ACADEMY OF SCIENCES OF USA, NATIONAL ACADEMY OF SCIENCE, WASHINGTON, DC, US, vol. 94, August 1997 (1997-08), pages 8405-8410, XP002064565 ISSN: 0027-8424
- IKEDA M ET AL: "Molecular cloning and characterization of a steroid receptor-binding regulator of G-protein signaling protein cDNA" GENE: AN INTERNATIONAL JOURNAL ON GENES AND GENOMES, ELSEVIER, AMSTERDAM, NL, vol. 273, no. 2, 8 August 2001 (2001-08-08), pages 207-214, XP004302704 ISSN: 0378-1119

## Description

This application claims priority from U.S. Provisional Application Serial No. 60/366,524, filed March 25, 2002.

### BACKGROUND OF THE INVENTION

### Field of the Invention

The present invention relates to methods of detecting nuclear receptor interactions. The methods of the invention are useful for detecting nuclear receptor (NR) activity, assaying NR activity, screening for NR ligands and cofactors (i.e., corepressors and coactivators), screening natural and surrogate ligands for orphan NRs, and screening compounds that interact with components of the NR regulatory process.

### Background of the Technology

Regulation of gene expression involves a large number of transcription factors with unique DNA-recognition properties. Many transcription factors belong to families of related proteins, the members of which bind to similar bu t distinct DNA sequences.

Nuclear receptors constitute a large family of ligand-activated transcription factors that interact with response elements within regulated genes. Nuclear receptors include receptors for steroid hormones, thyroid hormones, hormonal forms of vitamin A and D, peroxisomal activators, and ecdysone. Exemplary of steroid hormone receptors are estrogen receptor (ER) and progesterone receptor (PR). Other nuclear receptors include PPARγ (peroxisome proliferator activated receptor γ), RAR (retinoic acid receptor), RXR (retinoid X receptor), TR (thyroid hormone receptor) and VDR (vitamin D receptor).

Gene regulation by steroid hormones has been extensively studied. See, for example, Clever and Karlson, Exp. Cell Res., 20, 623 (1960). Additionally, steroid hormone receptors have been characterized and purified, hormonally regulated genes have been cloned, and hormone response sequences have been identified in the vicinity of genes regulated by steroid hormones. Further, dozens of regulatory elements for steroid hormones have been described, and the cDNAs for virtually all known hormone receptors have been cloned. See Evans, Science, 240, 889 (1988).

It has been widely proposed that steroid hormones mediate their biological responses by crossing the plasma membranes of cells and interacting with receptor proteins (i.e., steroid hormone or nuclear receptors) in the cytosol or nucleus of a cell to thereby form complexes. These ligand/receptor complexes then accumulate in the nucleus of cells where they bind to specific regulatory DNA sequences called hormone response elements (i.e., HREs). A dimer of the nuclear receptor/ligand complex is considered to bind to the appropriate response element, specifically to the core sequence of the response element. In this manner, the nuclear receptor/ligand complex can affect the transcription rate of dependent gene(s). Steroid hormone receptor/ligand complexes may also affect the stability of specific mRNAs.

Hormone response elements have been identified and characterized by several methods and have been shown to contain consensus sequences for the hormonal receptors. See, for example, Beato, Cell, 56, 335-344 (1989); Lopez de Haro, et al., FEBS Lett., 265, 20-22 (1990); and Baniahmad and Tsai, J. Cell Biochem, 51, 151-156 (1993).

Nuclear receptor complexes can bind to hormone response elements as homodimers and/or as heterodimers. Nuclear receptors that bind as homodimers include steroid receptors and retinoid X receptor. Nuclear receptors that bind as heterodimers include retinoic acid receptor, thyroid hormone receptor and vitamin D receptor. See for example, Moras, et al., "The Nuclear Receptor Ligand Bonding Domain: Structure and Function", Current Opinion in Cell Biology, 10, 384-391 (1998).

Nuclear receptors may also regulate gene expression via association with histone acetyltransferase (HAT) or deacetyltransferase complexes. See, for example, Chen, et al., "Regulation of Hormone-Induced Histone Hyperacetylation and Gene Activation via Acetylation of an Acetylase", Cell, Vol. 98 (1999). In particular, in the absence of their corresponding hormone, nuclear receptors are believed to repress the transcription of target genes via their association with corepressor complexes that contain histone deacetylase activity. Hormone binding is believed to trigger the release of these corepressors allowing for the subsequent association of an array of coactivators.

Various coactivators have been identified for nuclear receptors. These coactivators include p300/CBP, P/CAF (CBP associated factor), ACTR and SRC-1. These proteins have been demonstrated to interact with nuclear receptors and potentiate their transactivation activity. See Chen, et al. (1999), supra. ACTR, for example, has been found to form a multimeric activation complex with P/CAF and CBP/p300. See Chen, et al., "Nuclear Receptor Coactivator ACTR is a Novel Histone Acetyltransferase and Forms a Multimeric Activation Complex with P/CAF and CBP/p300", Cell, Vol. 90, 569-580 (1997).

Various nuclear receptor bioassays are known. United States Patent Nos. 5,071,773 and 5,298,429, for example, disclose bioassays for determining whether a protein suspected of being a hormone receptor has transcription-activating properties and for evaluating whether a compound is a functional ligand for receptor proteins. Additionally, United States Patent No. 5,770,176 discloses a method of detecting the presence or absence of functional nuclear receptors in a cell or tissue sample comprising simultaneously binding the nuclear receptor under assay occupied by its ligand to its associated response element and to an anti-receptor antibody.

There still exists a need, however, for bioassays which can be used to directly monitor protein-protein interactions between a nuclear receptor and a second protein such as a second nuclear receptor or a cofactor for the nuclear receptor. Such techniques would allow for the direct detection of protein-protein interactions *in situ* in a range of cell types and species. Such techniques could also be used to find ligands for orphan nuclear receptors by monitoring the interactions between an orphan nuclear receptor and a coactivator or a second nuclear receptor in the presence of a suspected ligand.

### SUMMARY OF THE INVENTION

According to a first aspect of the invention, a method of detecting nuclear receptor interactions is provided. The method includes providing a cell that expresses a first nuclear receptor as a fusion protein to a first fragment of a reporter molecule and a protein partner as a fusion protein to a second fragment of the reporter molecule and determining the presence and/or amount of the reporter molecule. The first and second fragments of the reporter molecule independently have no reporter function. However, the first and second fragments of the reporter molecule can interact to restore reporter function upon formation of a complex between the first nuclear receptor and the protein partner. The presence of the reporter molecule indicates formation of a complex between the nuclear receptor and the protein partner. The reporter molecule can be an enzyme (e.g., a monomeric or multimeric enzyme), a fluorescent protein, a luminescent protein, or a phosphorescent protein.

In one embodiment, the method includes providing a cell that expresses a first nuclear receptor as a fusion protein to a first inactive mutant form of a reporter enzyme. The cell also expresses a protein partner as a fusion protein to a second inactive mutant form of the reporter enzyme. The first and second inactive mutant forms of the reporter enzyme can interact upon formation of a complex between the first nuclear receptor and the protein partner to form an active reporter enzyme. The method according to this embodiment of the invention further includes determining the presence and/or amount of the reporter enzyme, wherein reporter enzyme activity in the cell indicates the formation of the complex between the nuclear receptor and the protein partner. The cell according to this embodiment of the invention can also contain a hormone response element operatively linked to a reporter gene such that binding of the nuclear receptor/protein partner complex to the hormone response element results in expression of the reporter gene. The presence and/or amount of a surrogate reporter protein encoded by the reporter gene can then be determined wherein the presence and/or amount of the surrogate reporter protein is an indication of the transcription-activating properties of the nuclear receptor/protein partner complex.

According to a second aspect of the invention, a DNA molecule comprising a sequence encoding a biologically active hybrid nuclear receptor is provided. The hybrid nuclear receptor comprises a nuclear receptor as a fusion protein to an inactive mutant form of a reporter enzyme.

According to a third aspect of the invention, a DNA molecule comprising a sequence encoding a biologically active hybrid of a nuclear receptor cofactor, wherein the hybrid cofactor comprises a nuclear receptor cofactor as a fusion protein to an inactive mutant form of a reporter enzyme is provided.

According to a fourth aspect of the invention, an isolated cell is provided wherein the cell comprises a first DNA construct capable of directing the expression of a first biologically active hybrid nuclear receptor in a cell and a second DNA construct capable of directing the expression of a biologically active protein partner in a cell. The first DNA construct includes a first promoter operatively linked to a first DNA molecule, the first DNA molecule comprising a sequence encoding a first biologically active nuclear receptor as a fusion protein to a first inactive mutant form of a reporter enzyme. The second DNA construct includes a second promoter operatively linked to a second DNA molecule, the second DNA molecule comprising a sequence encoding a biologically active protein partner as a fusion protein to a second inactive mutant form of the reporter enzyme. The first and second inactive mutant forms of the reporter enzyme can interact upon formation of a complex between the first nuclear receptor and the protein partner to form an active reporter enzyme.

According to a fifth aspect of the invention, a solid support having deposited thereon a plurality of cells is provided wherein the cells express a first nuclear receptor as a fusion protein to a first inactive mutant form of a reporter enzyme and a protein partner as a fusion protein to a second inactive mutant form of the reporter enzyme. The first and second inactive mutant forms of the reporter enzyme can interact to form an active reporter enzyme upon the formation of a complex between the first nuclear receptor and the protein partner.

### BRIEF DESCRIPTION OF THE DRAWINGS

The invention will be described with reference to the accompanying figures, wherein:

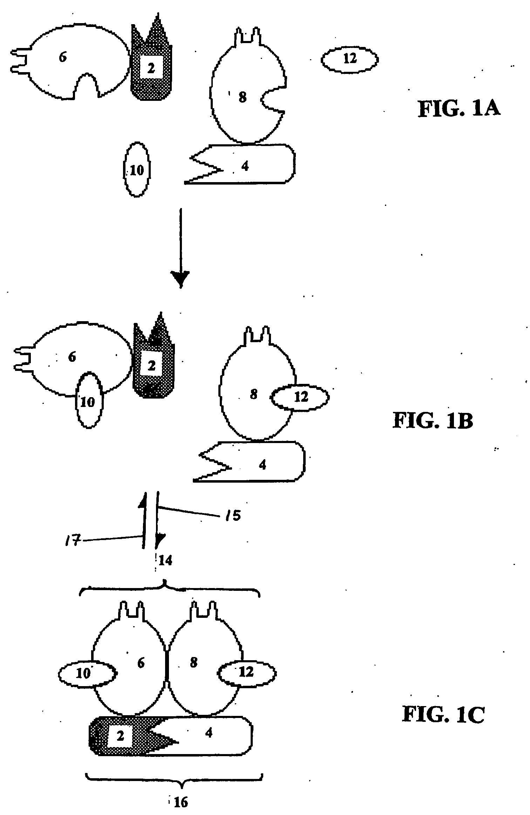

FIGS. 1A-1C illustrates a method of monitoring nuclear receptor dimerization according to the invention wherein a first nuclear receptor is fused to one complement enzyme fragment and a second NR is fused to a second complement enzyme fragment and wherein dimerization of the two NRs results in complementation of the enzyme fragments to produce an active enzyme complex;

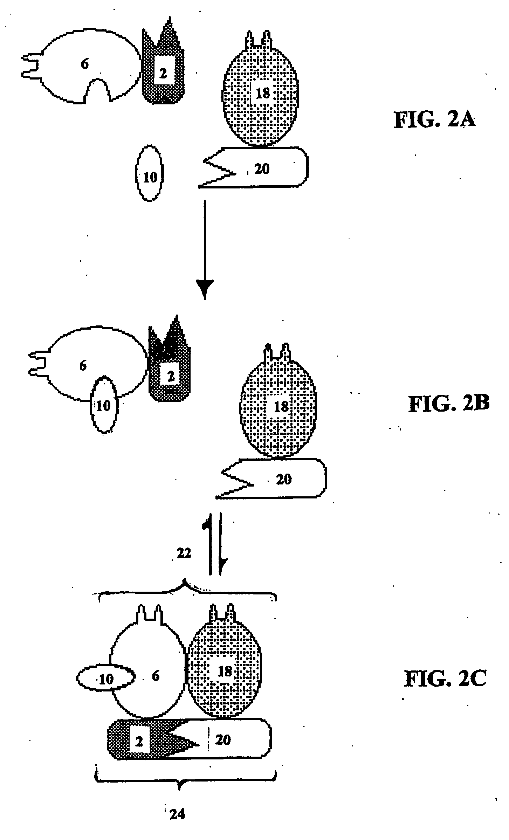

FIGS. 2A-2C illustrate the use of complementation technology in the method of the invention wherein two inactive mutant reporter enzymes become active upon the interaction of a nuclear receptor fused to a first galactosidase fragment and a cofactor for the nuclear receptor fused to a second galactosidase fragment;

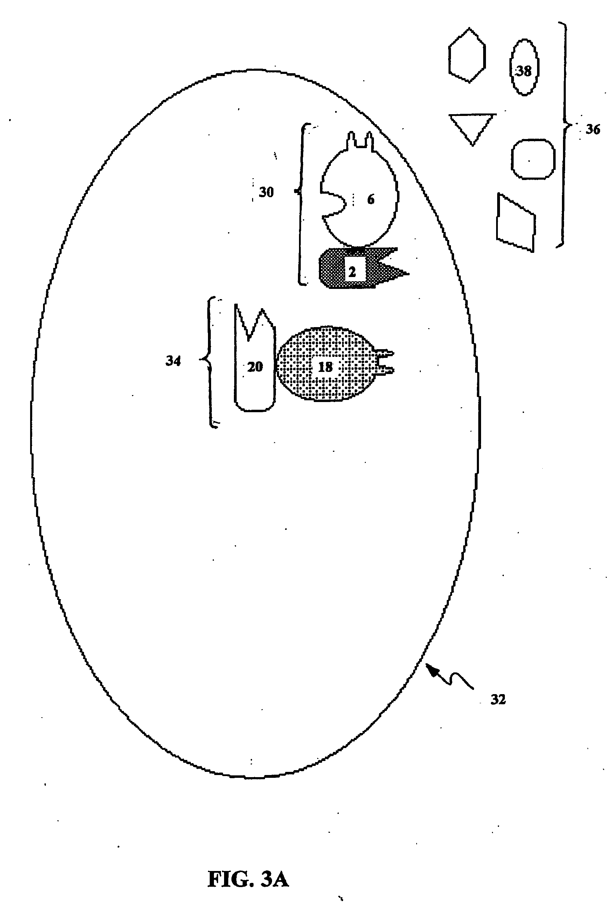

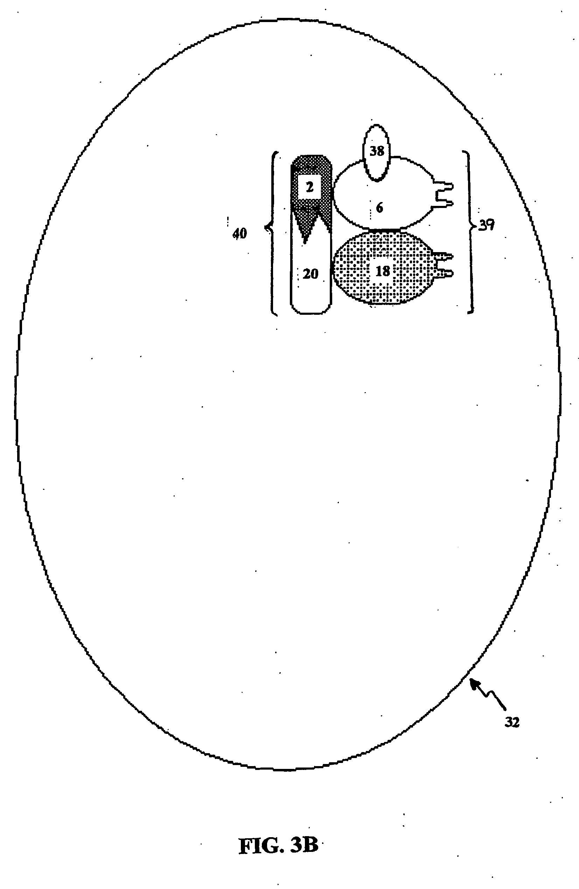

FIG. 3A and 3B illustrate a method for determining ligands (e.g. ligand fishing) for orphan nuclear receptors by β-galactosidase mutant complementation wherein a test cell expressing two β-gal fusion proteins (e.g., an orphan nuclear receptor fused to a first galactosidase fragment - NRₒᵣₚₕₐₙ-Δα) and a known cofactor (e.g., ACTR fused to a second galactosidase fragment - ACTR-Δω), is subjected to treatments with samples containing ligands;

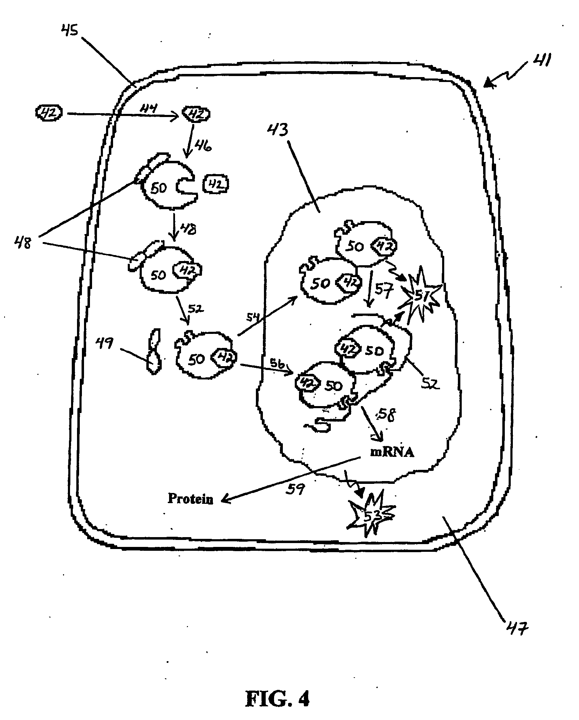

FIG. 4 illustrates Type I nuclear receptor complex formation (i.e., homodimerization) and gene expression in a cell wherein a ligand binds to the nuclear receptor in the cytoplasm activating the receptor for transport into the nucleus of the cell and binding to a hormone response element therein; and

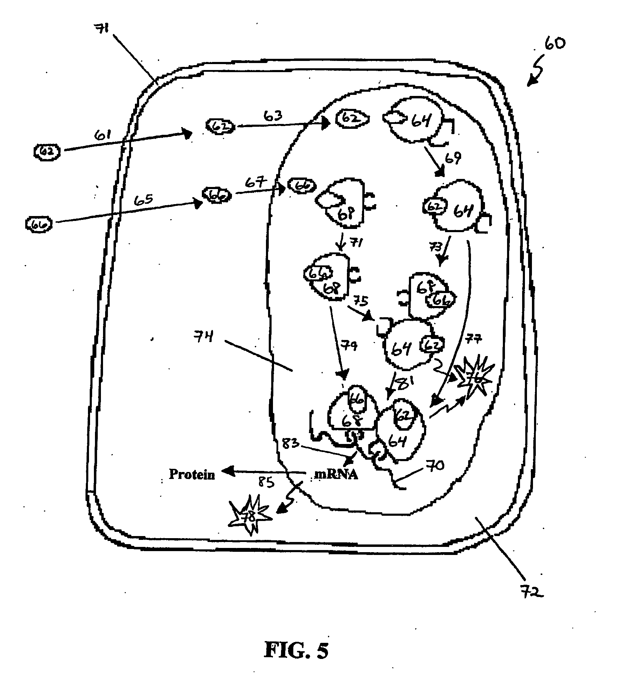

FIG. 5 illustrates Type II nuclear receptor complex formation (i.e., heterodimerization) and gene expression in a cell wherein ligands for each of the nuclear receptors are transported into the nucleus of the cell and bind to each of the nuclear receptors in the cell nucleus before dimerization and bonding to a hormone response element therein.

### DETAILED DESCRIPTION OF THE INVENTION

The present invention relates to protein-protein interaction assays for nuclear receptors. According to the invention, interactions between a nuclear receptor and a second protein can be monitored by reporter enzyme (e.g., β-galactosidase) complementation. The present invention provides a method to interrogate nuclear receptor function and pathways.

Only a fraction of nuclear receptors have been identified and even fewer have been associated with ligands (i.e., hormones). A ligand or hormone can be considered to be any small molecule that can bind to the receptor and affect its function. The means by which the identified orphan nuclear receptors and newly discovered orphan nuclear receptors will be associated with their cognate ligands and physiological functions represents a major challenge to biological and biomedical research.

The identification of an orphan nuclear receptor typically requires an individualized assay and a guess as to the function of the nuclear receptor. The present invention, however, involves the interrogation of nuclear receptor function by monitoring the activation of the receptor using activation dependent protein-protein interactions between the nuclear receptor and a second protein. The second protein can be a second nuclear receptor or a cofactor.

According to the invention, the specific protein-protein interactions can be measured using mutant enzyme complementation technology as described below. This assay system can eliminate guessing at the function of the nuclear receptor because it can be performed either with or without prior knowledge of other signaling events. Further, the assay system according to the invention is relatively sensitive and easy to perform. The assay system may also be applicable to nuclear receptors generically because many of the nuclear receptors are activated by a common mechanism. These generic mechanisms can be used, for example, to develop assays for orphan nuclear receptors.

According to the present invention, enzyme complementation technology can be used to monitor interactions between nuclear receptors and other proteins. Enzyme complementation technology is disclosed in U.S. Patent No. 6,893,827 (hereinafter the '827 patent), and U.S. Patent No. 6,342,345, issued January 29, 2002 (hereinafter the '345 patent), both of which are incorporated herein by reference in their entirety.

As described in the '827 patent and the '345 patent, enzyme complementation technology involves the use of two inactive enzyme (e.g., β-galactosidase) mutants, each of which is fused with one of two interacting target protein pairs. The formation of an active enzyme (e.g., β-galactosidase) complex is driven by the interaction of the target proteins. When the proteins of interest do not interact, the reporter enzyme remains inactive. When the proteins of interest do interact (e.g., when the proteins bind or dimerize), the reporter enzyme mutants come together and form an active enzyme.

Complementation techniques other than mutant enzyme complementation can also be used according to the invention. For example, protein fragment complementation assays can be used to measure the interactions between nuclear receptors and other proteins according to the invention. Protein fragment complementation assays are disclosed in U.S. Patent Nos.: 6,270,964; 6,294,330; and 6,428,951. Each of these patents is incorporated herein by reference in its entirety. Any of the protein fragment complementation assays disclosed in the aforementioned patents can be used to detect interactions between nuclear receptors and other proteins according to the invention.

According to an embodiment of the first aspect of the invention, a method of detecting nuclear receptor interactions using mutant enzyme complementation is provided. The method includes steps of: providing a cell that expresses a first nuclear receptor as a fusion protein to a first inactive mutant form of a reporter enzyme and a protein partner as a fusion protein to a second inactive mutant form of the reporter enzyme; and determining the presence and/or amount of the reporter enzyme. The first and second inactive mutant forms of the reporter enzyme can interact upon formation of a complex between the first nuclear receptor and the protein partner to form an active reporter enzyme. Reporter enzyme activity therefore indicates formation of a complex between the nuclear receptor and the protein partner. The protein partner according to the invention can be a wildtype protein or a mutant protein or any other regulatory protein, either known or unknown. For example, the protein partner can be a cofactor for the first nuclear receptor or a second nuclear receptor which can be the same or different than the first nuclear receptor. The co-factor can be a corepressor or a coactivator for the nuclear receptor. The cell can be a mammalian cell, a nematode cell, a yeast cell, a bacteria cell, or an insect cell.

According to a further embodiment of the first aspect of the invention, a method of detecting nuclear receptor interactions is provided using protein fragment complementation. The method includes providing a cell that expresses a first nuclear receptor as a fusion protein to a first fragment of a reporter molecule and a protein partner as a fusion protein to a second fragment of the reporter molecule and determining the presence and/or amount of the reporter molecule. The first and second fragments of the reporter molecule independently have no reporter function. However, the first and second fragments of the reporter molecule can interact to restore reporter function upon formation of a complex between the first nuclear receptor and the protein partner. The presence of the reporter molecule indicates formation of a complex between the nuclear receptor and the protein partner. The reporter molecule can be an enzyme (e.g., a monomeric or multimeric enzyme), a fluorescent protein, a luminescent protein, or a phosphorescent protein.

According to a further aspect of the invention, interactions between nuclear receptors and protein partners can be detected and quantitated *in vivo* (i.e., within isolated living cells) using the methods of the present invention. Vital enzyme substrates (e.g., vital β-gal substrates) which can be used in living cells are disclosed in U.S. Patent No. 6,342,345. Any of these vital substrates can be used according to the invention for *in vivo* detection.

Interactions between nuclear receptors and protein partners can also be detected and quantitated *in vitro* according to the invention. For example, according to one embodiment of the invention, the method as set forth above can further include lysing the cells and incubating the cell lysate with a substrate which emits a detectable signal after cleavage by the reporter enzyme. The substrate can be a chemiluminescent, colorimetric or fluorescent substrate. According to a preferred embodiment of the invention, the substrate is a chemiluminescent 1,2-dioxetane substrate.

According to a further aspect of the invention, the cell can also contain a hormone response element operatively linked to a reporter gene such that binding of a nuclear receptor complex to the hormone response element results in expression of the reporter gene. According to this aspect of the invention, the method further includes a step of determining the presence and/or amount of a surrogate reporter protein encoded by the reporter gene. The presence and/or amount of the surrogate reporter protein is therefore an indication of the transcription-activating properties of the nuclear receptor/protein partner complex. The surrogate reporter protein can be a surrogate reporter enzyme (e.g., luciferase) which is different than the reporter enzyme. The method according to this aspect of the invention can further include steps of lysing the cells and incubating the cell lysate with first and second substrates wherein the first substrate emits a first detectable signal after cleavage by the reporter enzyme and the second substrate emits a second detectable signal different than the first detectable signal after cleavage by the surrogate reporter enzyme. The first and second substrates can be chemiluminescent or fluorescent substrates.

According to a further aspect of the invention, the method can also include a step of exposing the cell to a compound comprising one or more ligands wherein increased reporter enzyme activity indicates agonist activity of the compound and decreased reporter enzyme activity indicates inverse agonist or antagonist activity of the compound. According to this aspect of the invention, the first nuclear receptor can be an orphan nuclear receptor.

According to a further aspect of the invention, a DNA molecule comprising a sequence encoding a biologically active hybrid nuclear receptor is provided wherein the hybrid nuclear receptor comprises a nuclear receptor as a fusion protein to an inactive mutant form of a reporter enzyme. The inactive mutant form of the reporter enzyme can be a β-galactosidase mutant. A DNA construct capable of directing the expression of the biologically active hybrid nuclear receptor in a cell is also provided. The DNA construct comprises a promoter operatively linked to a DNA molecule as set forth above. The inactive mutant form of the reporter enzyme in the DNA construct can be a β-galactosidase mutant. An isolated cell comprising a DNA construct as set forth above is also provided.

According to a further aspect of the invention, a DNA molecule comprising a sequence encoding a biologically active hybrid of a nuclear receptor cofactor is provided wherein the hybrid cofactor comprises a nuclear receptor cofactor as a fusion protein to an inactive mutant form of a reporter enzyme: The inactive mutant form of the reporter enzyme can be a β-galactosidase mutant. A DNA construct capable of directing the expression of a biologically active hybrid nuclear receptor cofactor in a cell is also provided. The DNA construct comprises a promoter operatively linked to a DNA molecule as set forth above. The inactive mutant form of the reporter enzyme in the DNA construct can be a β-galactosidase mutant. An isolated cell comprising a DNA construct as set forth above is also provided. According to a further embodiment of the invention, the nuclear receptor cofactor can be ACTR.

According to a further aspect of the invention, an isolated cell comprising a first DNA construct capable of directing the expression of a first biologically active hybrid nuclear receptor in a cell and a second DNA construct capable of directing the expression of a biologically active protein partner in a cell is provided. The first DNA construct comprises a first promoter operatively linked to a first DNA molecule comprising a sequence encoding a first biologically active nuclear receptor as a fusion protein to a first inactive mutant form of a reporter enzyme. The second DNA construct comprises a second promoter operatively linked to a second DNA molecule comprising a sequence encoding a biologically active protein partner as a fusion protein to a second inactive mutant form of the reporter enzyme. The first and second inactive mutant forms of the reporter enzyme can interact upon formation of a complex between the first nuclear receptor and the protein partner to form an active reporter enzyme. The protein partner can be a cofactor for the first nuclear receptor or a second nuclear receptor which can be the same as or different than the first nuclear receptor.

According to a further aspect of the invention, a solid support having a plurality of cells deposited thereon is provided. According to this aspect of the invention, the cells express a first nuclear receptor as a fusion protein to a first inactive mutant form of a reporter enzyme and a protein partner as a fusion protein to a second inactive mutant form of the reporter enzyme. The first and second inactive mutant forms of the reporter enzyme can interact to form an active reporter enzyme upon the formation of a complex between the first nuclear receptor and the protein partner. According to this aspect of the invention, the cells can further comprise an enzyme substrate comprising an enzyme-labile group which, upon cleavage by the reporter enzyme, emits a detectable signal. The signal can be a colorimetric, fluorescent or chemiluminescent signal. The solid support can be made of glass, plastic, ceramic, semiconductor, silica, fiber optic, diamond, and bio-compatible materials (e.g., bio-compatible monomers or polymers).

According to a preferred embodiment of the invention, a first inactive β-galactosidase mutant is fused to a first nuclear receptor and a second β-galactosidase mutant is fused to second protein (i.e., protein partner). The second protein can interact with the first nuclear receptor. The first and second inactive β-galactosidase mutants can form an active enzyme when the first nuclear receptor and the second protein interact to form a complex. The second protein can be a second nuclear receptor (which may be the same or different than the first nuclear receptor) or a cofactor for the first nuclear receptor.

FIGS. 1A-1C illustrate the use of β-galactosidase complementation technology according to the invention wherein two inactive β-galactosidase mutants 2, 4 (e.g., Δα and Δω) become active 16 when the protein fusion partners of the two inactive β-galactosidase mutants 6, 8 interact to form a dimer 14. As shown in FIGS. 1A-1C, these protein fusion partners 6, 8 are first and second nuclear receptors (e.g., NR₁ and NR₂) each of which are activated by their respective ligands 10, 12. FIG. 1A shows protein fusion partners 6, 8 (i.e., the nuclear receptors) before ligand activation, FIG. 1B shows the nuclear receptors 6, 8 associated with their respective ligands 10, 12 and FIG. 1C shows receptors 6, 8 after formation of the dimer 14. According to the invention, the active β-galactosidase 16 resulting from the interaction of the two protein fusion partners 6, 8 can cleave an enzyme labile group on a chemiluminescent substrate to produce light.

The first and second nuclear receptors 6, 8 shown in FIGS. 1A-1C can be the same, in which case homo-dimer formation can be monitored. Alternatively, the first and second nuclear receptors 6, 8 can be different, in which case heterodimer formation can be monitored. As shown in FIGS. 1B and 1C, each of the nuclear receptors 6, 8 are associated with ligands 10, 12 respectively. Nuclear receptor dimer formation, which is illustrated in FIG. 1C, may be ligand activated in which case increased chemiluminescence can be observed if a ligand for the nuclear receptor is present. As indicated in FIGS. 1A-1C, agonist activity 15 promotes dimer formation whereas antagonist or inverse agonist activity 17 promotes monomer formation. According to the invention, the agonist or antagonist activity of various compounds on nuclear receptor complex formation can be monitored.

FIGS. 2A-2C illustrate the use of complementation technology according to the invention wherein two inactive β-galactosidase mutants 2, 20 (e.g., Δα and Δω) become active 24 when the protein fusion partners (i.e., a nuclear receptor and a co-factor) of the two inactive β-galactosidase mutants 6, 18 interact to form a dimer 22. In FIGS. 2A-2C, a nuclear receptor 6 is shown as a fusion protein with a first β-galactosidase mutant 2 (e.g., Δα), and a cofactor 18 (e.g., ACTR) is shown as a fusion protein with a second β-galactosidase mutant 20 (e.g., Δω). FIG. 2A shows the nuclear receptor 6 before activation by ligand 10, FIG. 2B shows the ligand activated receptor 6, and FIG. 2C shows the nuclear receptor-cofactor complex 22.

After enzyme complementation, enzyme activity according to the invention can be measured by enzyme activity assays according to techniques known in the art. Increased β-galactosidase activity can be an indication that the receptor and the cofactor have interacted to form a complex.

Nuclear receptor assays according to the invention can also be used to determine ligands for orphan nuclear receptors. This process is commonly referred to as a "de-orphaning" or a "ligand fishing" process. A procedure of this type is illustrated in FIGS. 3A and 3B wherein a β-galactosidase fusion protein 30 of an orphan receptor 6 (e.g., NRₒ-Δα) is co-expressed in a test cell 32 with a fusion protein 34 of a known cofactor 18 (e.g., ACTR-Δω) for the nuclear receptor. In FIGS. 3A and 3B, the β-galactosidase mutants fused to orphan receptor 6 and cofactor 18 are denoted by reference numerals 2 and 18 respectively.

As shown in FIG. 3B, when test cell 32 is subjected to compounds containing various ligands 36, including the ligand 38 for the receptor 6, ligand activation of the nuclear receptor 6 can result in the formation of a receptor-cofactor complex 39. According to the invention, formation of complex 39 can produce an increase in β-galactosidase 40 activity which can be used to indicate that the compound contains either a natural or surrogate ligand for the nuclear receptor.

The technique illustrated in FIGS. 3A and 3B can also be practiced with a second nuclear receptor capable of dimerizing with the orphan nuclear receptor rather than with a cofactor for the orphan nuclear receptor.

The above assay technique can be used to find and develop potential drugs for orphan nuclear receptors. For example, increased β-galactosidase activity in the test cell after treatment with a compound can indicate agonist activity of the compound. Alternatively, decreased β-galactosidase activity in the test cell can indicate antagonist activity or inverse agonist activity of the compound.

Also according to the invention, the complementation technology for measuring nuclear receptor complex formation can be combined with the use of a surrogate reporter (e.g., luciferase) for monitoring the effects of nuclear receptor complex formation on gene expression. Reporter gene assays are widely used in the art to measure the activity of a gene's promoter. This technique takes advantage of molecular biology techniques in which heterologous genes under the control of any promoter are introduced into the genome of a mammalian cell. See, for example, Gorman, et al., Mol. Cell Biol. 2, 1044-1051 (1982); and Alam, et al., Anal. Biochem. 188, 245-254 (1990). Activation of the promoter induces the expression of the reporter gene. By design, the reporter gene codes for a reporter protein that can easily be detected and measured. The reporter protein is typically a reporter enzyme that can convert a substrate (e.g., a fluorescent or a chemiluminescent substrate) into a product. This conversion can be conveniently followed by direct optical measurement and can therefore allow for the quantification of the amount of reporter enzyme activity produced.

Gene expression according to the invention can also be monitored using other assay technology as known in the art. For example, mRNA can be quantified using a reverse transcription polymerase chain reaction assay. An assay of this type is the "TaqMan" assay which utilizes the 5' nuclease activity of the DNA polymerase to hydrolyze a hybridization probe bound to its target amplicon. "TaqMan" is a registered trademark of Hoffman-La-Roche, Inc. The "TaqMan" assay and other methods of quantifying mRNA are reviewed in Bustin, Absolute Quantification of mRNA using Real Time Reverse Transcription Polymerase Chain Reaction Assays, J. of Mol. Endocrinology, 25, 169-193 (2000).

FIGS. 4 and 5 illustrate how both nuclear receptor complex formation (e.g., dimerization) and the downstream effects of complex formation on gene expression can be monitored in a recombinant cell line. In FIGS. 4 and 5, a β-galactosidase fusion protein of a nuclear receptor is co-expressed in a test cell with a fusion protein of a second receptor. In FIG. 4, the second receptor is the same as the first receptor whereas, in FIG. 5, the second receptor is different than the first receptor. The test cell can also contain a DNA sequence encoding a hormone response element operatively linked to a reporter gene. DNA sequences of this type are described in United States Patent Nos. 5,071,773 and 5,298,429, which are hereby incorporated by reference in their entirety.

FIG. 4 illustrates dimerization and gene expression for Type I nuclear receptors. It is known that Type I ligands (e.g., cortisol, testosterone, etc.) and estrogen (i.e., estradiol) bind to their corresponding nuclear receptors in the cytosol. As shown in FIG. 4, a ligand 42 is transferred 44 through the plasma membrane 45 and into the cytoplasm 47 of a cell 41. As shown in FIG. 4, the cell 41 contains a nuclear receptor 50 associated with a heat shock protein 48. Once ligand 42 binds 46, 48 to nuclear receptor 50, nuclear receptor 50 dissociates 52 from heat shock protein 49 and becomes "activated" for binding to a hormone response element. The activated receptor can then move 54, 56 into the nucleus 43 of the cell 41. Once inside the nucleus 43, the activated receptor either dimerizes then binds 54, 57 or binds sequentially 56 to the corresponding hormone response element (HRE) 52. As a result, the transcription 58 of the particular DNA to which the dimer has bound can be regulated. The transcribed messenger RNA in the nucleus 43 can then move 59 into the cytosol 47 where it can be translated on ribosomes into a protein.

According to the invention, the protein-protein interactions 54, 56 can be used to produce a first signal 51 and gene expression 58, 59 can be used to produce a second signal 53. The second signal 53 can be generated from the expression of a reporter gene. In this manner, the nuclear receptor interactions and the downstream effects of these interactions on gene expression can be monitored.

FIG. 5 illustrates dimerization and gene expression for Type II nuclear receptors. Ligands for Type II nuclear receptors include vitamin D, thyroid hormones (T3) and retinoids (Vitamin A). Activation for a Type II receptor can lead to either homo- or hetero-dimerization (which is shown in FIG. 5) and then DNA binding. In the case of the retinoic acid receptor (RAR), for example, the DNA response element binds two receptors of the same type (homodimer) in the presence of all-trans retinoic acid ligand and represses DNA transcription. In the presence of T3 (e.g., triiodiothyronine), however, one receptor for T3 exchanges with one receptor of bound RAR to produce an RAR/T3 receptor heterodimer. This heterodimer turns on the DNA transcription. The effect an activated receptor has on its target DNA can be dependent upon a variety of factors including the relationship of its hormone response elements to other DNA elements as well as the transcription factors for that particular DNA.

As shown in FIG. 5, ligands 62, 66 move 61, 65 through plasma membrane 71 and cytoplasm 72 and into nucleus 74 of a cell 60 without binding any receptors. Once in the nucleus, ligands 62, 66 can activate receptors 64, 68. The ligand activated receptors can then either bind sequentially 77, 79 to the hormone response element 70 or dimerize first 73, 75 and then bind in dimer form 81 to hormone response element 70. As a result, the transcription of messenger RNA (i.e., mRNA) can be effected. As shown in FIG. 5, the transcribed messenger RNA in the nucleus can then move 85 into the cytosol 72 where it can be translated on ribosomes into a protein. According to the invention, the protein-protein interactions 73, 75 or 77, 79 can be used to produce a first signal 76 and gene expression 83, 85 can be used to produce a second signal 78. In this manner, both nuclear receptor interactions and the downstream effects of these interactions on gene expression can be monitored.

According to the invention, the transcribed mRNA can be assayed using various techniques known in the art. In this manner, the effects of protein-protein interactions on gene expression can be monitored. Alternatively, a reporter gene (e.g., luciferase) linked to a promoter (e.g., the regulatory sequences of a particular hormone response element) can be used to determine the effects of protein-protein interactions on gene expression. In this manner, the influence of protein-protein interactions on expression of the reporter gene can be related to expression of the gene of interest.

FIGS. 4 and 5 are merely intended to illustrate various factors that may be present and which may interact within the cell. These figures and the discussion thereof are not intended to limit the invention in any way.

According to the invention, β-galactosidase activity resulting from protein-protein interactions can be detected using an assay system wherein cell lysis is combined with chemiluminescent detection of β-galactosidase reporter enzyme activity. The assay system according to the invention can, for example, employ a chemiluminescent substrate such as a 1,2-dioxetane compound having an enzyme labile substituent and one or more stabilizing groups. Chemiluminescent substrates of this type are disclosed, for example, in U.S. Patent Nos. 5,851,771; 5,538,847; 5,326,882; 5,145,772; 4,978,614 and 4,931,569, the contents of each of which are incorporated herein by reference in their entirety. Any of the chemiluminescent substrates disclosed in the aforementioned references can be used in assays according to the invention. Other chemiluminescent substrates can also be used. Any substrate which emits light upon enzyme cleavage can be used according to the invention. According to a preferred embodiment of the invention, the chemiluminescent substrate is a 1,2-dioxetane having an adamantyl stabilizing group. A material of this type is available under the trademark Galacton-*Star*^{®}, which is a registered trademark of Applera Corporation or its subsidiaries.

Light output resulting from enzymatic activity according to the invention can be measured in a luminometer to thereby provide a measure of nuclear receptor complex formation as well as the effects of complex formation on gene expression. For example, β-galactosidase reporter enzyme activity can provide a measure of nuclear receptor complex formation whereas the enzyme activity of a surrogate reporter (e.g., luciferase) can provide a measure of gene expression. Light output according to the invention can also be measured using a scintillation counter or any other known light measuring device.

An assay according to the invention can be performed by incubating the cell lysate with a reaction buffer containing the chemiluminescent substrate and, optionally, a chemiluminescent substrate enhancer. Any known chemiluminescent substrate enhancer can be used according to the invention. Incubation can be conducted until maximum light emission is reached at which point light output can be measured. An assay system of this type is available under the trademark Galacto-*Star*^{™}, which is a trademark of Applera Corporation or its subsidiaries. Other assay systems and techniques, however, can also be used according to the invention.

As set forth above, the invention is achieved in part by using protein/protein interaction screening to map signaling pathways. This technology can be used with known and unknown nuclear receptors having diverse functions including orphan nuclear receptors wherein the natural ligand to the receptor has not been identified.

Use of galactosidase complementation technology provides many benefits to the nuclear receptor screening process, including the ability to monitor protein interactions in any sub-cellular compartment membrane (e.g., cytosol or nucleus). Moreover, the present invention provides nuclear receptor binding assays that can be achieved directly within the cellular environment in a rapid, non-radioactive assay format. The assay techniques of the present invention can therefore provide a more physiologically relevant model without the need for protein over-expression. The assay system of the invention can also provide a cell-based method for interrogating nuclear receptor pathways which is amenable to high-throughput screening (HTS).

The present invention has numerous additional advantages. First, it is applicable to a variety of cells including mammalian cells, nematode cells, yeast cells, bacterial cells and insect cells. Second, it can detect interactions in the cytosol or nucleus of a cell. Also, it does not rely on indirect read-outs such as transcriptional activation but, rather, allows interactions between a nuclear receptor and a second protein to be directly monitored. The present invention can thus provide assays with a high degree of physiological relevance.

The methods of the present invention can be used to determine the effects of mutations on the interactions between a nuclear receptor and a second protein. For example, the effect of single nucleotide polymorphisms (i.e., SNPs), such as coding SNPs, on interactions between nuclear receptors and other proteins can be determined according to the invention. Coding SNPs are SNPs which alter the sequence of the protein encoded by the altered or mutated gene. According to the invention, interactions between SNPs of nuclear receptors and protein partners (e.g., cofactors) can be determined. Alternatively, interactions between nuclear receptors and SNPs of protein partners (e.g., cofactors) can also be determined according to the invention. Additionally, the effect of various mutations of nuclear receptors and/or protein partners (e.g., cofactors) on the interaction between the two proteins can be determined according to the invention.

The assays of this invention, and their application and preparation have been described both generically and by specific example. The examples, however, are not intended to be limiting. Other embodiments will occur to those of ordinary skill in the art without the exercise of inventive faculty. Such modifications remain within the scope of the invention.

## Claims

1. An in vitro method of detecting nuclear receptor interactions, the method comprising:
providing a cell that expresses: a first nuclear receptor as a fusion protein to a first fragment of a reporter molecule; and a protein partner as a fusion protein to a second fragment of the reporter molecule, wherein the first and second fragments of the reporter molecule independently have no reporter function and wherein the first and second fragments of the reporter molecule interact to restore reporter function upon formation of a complex between the first nuclear receptor and the protein partner; and
determining the presence and/or amount of the reporter molecule;
wherein the presence of the reporter molecule indicates formation of a complex between the nuclear receptor and the protein partner.

2. The method of Claim 1, wherein the reporter molecule is selected from the group consisting of a monomeric enzyme, a multimeric enzyme, a fluorescent protein, a luminescent protein, and a phosphorescent protein.

3. The method of claim 1, wherein the first fragment of the reporter molecule is a first inactive mutant form of a reporter enzyme and wherein the second fragment of the reporter molecule is a second inactive mutant form of the reporter enzyme and wherein the first and second inactive forms of the reporter enzyme interact upon formation of a complex between the first nuclear receptor and the protein partner to form an active reporter enzyme.

4. The method of any one of Claims 1 to 3, wherein the protein partner is a cofactor for the first nuclear receptor.

5. The method of Claim 4, wherein the cofactor is a corepressor or a coactivator.

6. The method of Claim 4, wherein the cofactor is a wildtype protein or a mutant protein.

7. The method of Claim 4, wherein reporter enzyme activity is detected in vitro in a living cell.

8. The method of any one of Claims 1 to 3, wherein the protein partner is a second nuclear receptor.

9. The method of Claim 8, wherein the second nuclear receptor is the same as the first nuclear receptor.

10. The method of Claim 8, wherein the second nuclear receptor is different than the first nuclear receptor.

11. The method of claim 3, wherein the cell also contains a hormone response element operatively linked to a reporter gene such that binding of the nuclear receptor/protein partner complex to the hormone response element results in expression of the reporter gene, the method further comprising:
determining the presence and/or amount of a surrogate reporter protein encoded by the reporter gene;
wherein the presence and/or amount of the surrogate reporter protein is an indication of the transcription-activating properties of the nuclear receptor/protein partner complex.

12. The method of Claim 11, wherein the surrogate reporter protein is a surrogate reporter enzyme which is different than the reporter enzyme.

13. The method of Claim 12, wherein the surrogate reporter enzyme is luciferase.

14. The method of Claim 3, further comprising:
exposing the cell to a compound comprising one or more ligands;
wherein increased reporter enzyme activity indicates agonist activity of the compound and decreased reporter enzyme activity indicates inverse agonist or antagonist activity of the compound.

15. The method of Claim 14, wherein the first nuclear receptor is an orphan nuclear receptor.

16. The method of claim 3, further comprising:
lysing the cells; and
incubating the cell lysate with a substrate, wherein the substrate emits a detectable signal after cleavage by the reporter enzyme.

17. The method of Claim 16, wherein the substrate is a chemiluminescent or fluorescent substrate.

18. The method of Claim 16, wherein the substrate is a chemiluminescent 1,2-dioxetane substrate.

19. The method of Claim 12, further comprising:
lysing the cells; and
incubating the cell lysate with first and second substrates, wherein the first substrate emits a first detectable signal after cleavage by the reporter enzyme and
wherein the second substrate emits a second detectable signal different than the first detectable signal after cleavage by the surrogate reporter enzyme.

20. The method of Claim 19, wherein the first and second substrates are independently selected from the group consisting of chemiluminescent and fluorescent substrates.

21. The method of anyone of Claims 1 to 3, wherein the cell is selected from the group consisting of mammalian cells, nematode cells, insect cells, yeast cells and bacteria cells.

22. A DNA molecule comprising a sequence encoding a biologically active hybrid nuclear receptor, wherein the hybrid nuclear receptor comprises a nuclear receptor as a fusion protein to an inactive mutant form of a reporter enzyme.

23. The DNA molecule of Claim 22, wherein the inactive mutant form of the reporter enzyme is a β-galactosidase mutant.

24. A DNA construct capable of directing the expression of a biologically active hybrid nuclear receptor in a cell, the DNA construct comprising the following operatively linked elements:
a promoter; and
the DNA molecule of Claim 22.

25. The DNA construct of Claim 24, wherein the inactive mutant form of the reporter enzyme is a β-galactosidase mutant.

26. An isolated cell comprising the DNA construct of Claim 24.

27. An isolated cell comprising the DNA construct of Claim 25.

28. A DNA molecule comprising a sequence encoding a biologically active hybrid of a nuclear receptor cofactor as a fusion protein to an inactive mutant form of a reporter enzyme.

29. The DNA molecule of Claim 28, wherein the inactive mutant form of the reporter enzyme is a β-galactosidase mutant.

30. A DNA construct capable of directing the expression of a biologically active hybrid of a nuclear receptor cofactor in a cell, comprising the following operatively linked elements:
a promoter; and
the DNA molecule of Claim 28.

31. The DNA construct of Claim 30, wherein the inactive mutant form of the reporter enzyme is a β-galactosidase mutant.

32. An isolated cell comprising the DNA construct of Claim 30.

33. An isolated cell comprising the DNA construct of Claim 31.

34. The DNA molecule of Claim 28, wherein the nuclear receptor cofactor is ACTR.

35. An isolated cell comprising:
a first DNA construct capable of directing the expression of a first biologically active hybrid nuclear receptor in a cell, the first DNA construct comprising a first promoter operatively linked to a first DNA molecule, the first DNA molecule comprising a sequence encoding a first biologically active nuclear receptor as a fusion protein to a first inactive mutant form of a reporter enzyme; and
a second DNA construct capable of directing the expression of a biologically active protein partner in a cell, the second DNA construct comprising a second promoter operatively linked to a second DNA molecule, the second DNA molecule comprising a sequence encoding a biologically active protein partner as a fusion protein to a second inactive mutant form of the reporter enzyme;
wherein the first and second inactive mutant forms of the reporter enzyme interact upon formation of a complex between the first nuclear receptor and the protein partner to form an active reporter enzyme.

36. The cell of Claim 35, wherein the protein partner is a cofactor for the first nuclear receptor.

37. The cell of Claim 35, wherein the protein partner is a second nuclear receptor.

38. The cell of claim 37, wherein the second nuclear receptor is the same as the first nuclear receptor.

39. The cell of Claim 37, wherein the second nuclear receptor is different than the first nuclear receptor.

40. A solid support having deposited thereon a plurality of cells, wherein the cells express:
a first nuclear receptor as a fusion protein to a first inactive mutant form of a reporter enzyme; and
a protein partner as a fusion protein to a second inactive mutant form of the reporter enzyme;
wherein first and second inactive mutant forms of the reporter enzyme interact to form an active reporter enzyme upon the formation of a complex between the first nuclear receptor and the protein partner.

41. A solid support according to Claim 40, wherein the cells comprise an enzyme substrate comprising an enzyme-labile chemical group which, upon cleavage by the reporter enzyme, releases a product measurable by colorimetry, fluorescence or chemiluminescence.

42. A solid support according to Claim 40, wherein the solid support is made of a material selected from the group consisting of glass, plastic, ceramic, semiconductor, silica, fiber optic, diamond, bio-compatible monomers and bio-compatible polymer materials.

## Patentansprüche

1. In vitro-Verfahren zum Nachweis von Wechselwirkungen eines nukleären Rezeptors, wobei das Verfahren umfasst:
Bereitstellen einer Zelle, die einen ersten nukleären Rezeptor als Fusionsprotein an ein erstes Fragment eines Reportermoleküls und einen Proteinpartner als ein Fusionsprotein an ein zweites Fragment des Reportermoleküls exprimiert, wobei die ersten und zweiten Fragmente des Reportermoleküls unabhängig keine Reporterfunktion aufweisen und die ersten und zweiten Fragmente des Reportermoleküls bei Bildung eines Komplexes zwischen dem ersten nukleären Rezeptor und dem Proteinpartner wechselwirken, um eine Reporterfunktion wiederherzustellen, und
Bestimmen des Vorliegens und/oder der Menge des Reportermoleküls,
wobei das Vorliegen des Reportermoleküls eine Bildung eines Komplexes zwischen dem nukleären Rezeptor und dem Proteinpartner anzeigt.

2. Verfahren nach Anspruch 1, wobei das Reportermolekül ausgewählt ist aus der Gruppe, bestehend aus einem monomerischen Enzym, einem multimerischen Enzym, einem fluoreszierenden Protein, einem lumineszierenden Protein und einem phosphoreszierenden Protein.

3. Verfahren nach Anspruch 1, wobei das erste Fragment des Reportermoleküls eine erste inaktive mutierte Form eines Reporterenzyms ist und das zweite Fragment des Reportermoleküls eine zweite inaktive mutierte Form des Reporterenzyms ist und wobei die ersten und zweiten inaktiven Formen des Reporterenzyms bei Bildung eines Komplexes zwischen dem ersten nukleären Rezeptor und dem Proteinpartner wechselwirken, um ein aktives Reporterenzym auszubilden.

4. Verfahren nach einem der Ansprüche 1-3, wobei der Proteinpartner ein Co-Faktor für den ersten nukleären Rezeptor ist.

5. Verfahren nach Anspruch 4, wobei der Co-Faktor ein Co-Repressor oder ein Co-Aktivator ist.

6. Verfahren nach Anspruch 4, wobei der Co-Faktor ein Wildtyp-Protein oder ein mutiertes Protein ist.

7. Verfahren nach Anspruch 4, wobei Reporterenzym-Aktivität in vitro in einer lebenden Zelle nachgewiesen wird.

8. Verfahren nach einem der Ansprüche 1-3, wobei der Proteinpartner ein zweiter nukleärer Rezeptor ist.

9. Verfahren nach Anspruch 8, wobei der zweite nukleäre Rezeptor zu dem ersten nukleären Rezeptor identisch ist.

10. Verfahren nach Anspruch 8, wobei der zweite nukleäre Rezeptor zu dem ersten nukleären Rezeptor verschieden ist.

11. Verfahren nach Anspruch 3, wobei die Zelle auch ein Hormonresponseelement enthält, das operabel mit einem Reportergen verbunden ist, so dass eine Bindung des Komplexes aus nukleärem Rezeptor und Proteinpartner an das Hormonresponseelement zu einer Expression des Reportergens führt, wobei das Verfahren ferner umfasst:
Bestimmen des Vorliegens und/oder der Menge eines durch das Reportergen kodierten Surrogat-Reporterproteins,
wobei das Vorliegen und/oder die Menge des Surrogat-Reporterproteins ein Anzeichen für die transkriptionsaktivierenden Eigenschaften des Komplexes aus nukleärem Rezeptor und Proteinpartner ist.

12. Verfahren nach Anspruch 11, wobei das Surrogat-Reporterprotein ein Surrogat-Reporterenzym ist, das zu dem Reporterenzym verschieden ist.

13. Verfahren nach Anspruch 12, wobei das Surrogat-Reporterenzym Luciferase ist.

14. Verfahren nach Anspruch 3, das ferner umfasst:
Exponieren der Zelle gegenüber einer Verbindung, die einen oder mehrere Liganden umfasst,
wobei eine erhöhte Reporterenzym-Aktivität eine Agonisten-Aktivität der Verbindung anzeigt und eine verringerte Reporterenzym-Aktivität eine inverse Agonisten- oder Antagonistenaktivität der Verbindung anzeigt.

15. Verfahren nach Anspruch 14, wobei der erste nukleäre Rezeptor ein Orphan-nukleärer Rezeptor ist.

16. Verfahren nach Anspruch 3, das ferner umfasst:
Lysieren der Zellen und Inkubieren des Zell-Lysats mit einem Substrat,
wobei das Substrat ein nachweisbares Signal nach Spaltung durch das Reporterenzym aussendet.

17. Verfahren nach Anspruch 16, wobei das Substrat ein chemilumineszierendes oder fluoreszierendes Substrat ist.

18. Verfahren nach Anspruch 16, wobei das Substrat ein chemilumineszierendes 1,2-Dioxethansubstrat ist.

19. Verfahren nach Anspruch 12, das ferner umfasst:
Lysieren der Zellen und Inkubieren des Zell-Lysats mit ersten und zweiten Substraten, wobei das erste Substrat ein erstes nachweisbares Signal nach Spaltung durch das Reporterenzym aussendet und das zweite Substrat ein zweites nachweisbares Signal, das zu dem ersten nachweisbaren Signal verschieden ist, nach Spaltung durch das Surrogat-Reporterenzym aussendet.

20. Verfahren nach Anspruch 19, wobei die ersten und zweiten Substrate unabhängig voneinander ausgewählt sind aus der Gruppe, bestehend aus chemilumineszierenden und fluoreszierenden Substraten.

21. Verfahren nach einem der Ansprüche 1-3, wobei die Zelle ausgewählt ist aus der Gruppe, bestehend aus Säugerzellen, Nematodenzellen, Insektenzellen, Hefezellen und Bakterienzellen.

22. DNA-Molekül, das eine Sequenz umfasst, die für einen biologisch wirksamen hybriden nukleären Rezeptor kodiert, wobei der hybride nukleäre Rezeptor einen nukleären Rezeptor als ein Fusionsprotein an eine inaktive mutierte Form eines Reporterenzyms umfasst.

23. DNA-Molekül nach Anspruch 22, wobei die inaktive mutierte Form des Reporterenzyms eine β-Galactosidase-Mutante ist.

24. DNA-Konstrukt, das zum Steuern der Expression eines biologisch wirksamen hybriden nukleären Rezeptors in einer Zelle fähig ist, wobei das DNA-Konstrukt die nachstehenden operabel verbundenen Elemente umfasst: einen Promotor und das DNA-Molekül nach Anspruch 22.

25. DNA-Konstrukt nach Anspruch 24, wobei die inaktive mutierte Form des Reporterenzyms eine β-Galactosidase-Mutante ist.

26. Isolierte Zelle, die das DNA-Konstrukt nach Anspruch 24 umfasst.

27. Isolierte Zelle, die das DNA-Konstrukt nach Anspruch 25 umfasst.

28. DNA-Molekül, das eine Sequenz umfasst, die für ein biologisch wirksames Hybrid eines Co-Faktors eines nukleären Rezeptors als Fusionsprotein an eine inaktive mutierte Form eines Reporterenzyms kodiert.

29. DNA-Molekül nach Anspruch 28, wobei die inaktive mutierte Form des Reporterenzyms eine β-Galactosidase-Mutante ist.

30. DNA-Konstrukt, das zum Steuern der Expression eines biologisch wirksamen Hybrids eines Co-Faktors eines nukleären Rezeptors in einer Zelle fähig ist, umfassend die nachstehenden operabel verbundenen Elemente: einen Promotor und das DNA-Molekül nach Anspruch 28.

31. DNA-Konstrukt nach Anspruch 30, wobei die inaktive mutierte Form des Reporterenzyms eine β-Galactosidase-Mutante ist.

32. Isolierte Zelle, die das DNA-Konstrukt nach Anspruch 30 umfasst.

33. Isolierte Zelle, die das DNA-Konstrukt nach Anspruch 31 umfasst.

34. DNA-Molekül nach Anspruch 28, wobei der Co-Faktor des nukleären Rezeptors ACTR ist.

35. Isolierte Zelle, die umfasst:
ein erstes DNA-Konstrukt, das zum Steuern der Expression eines ersten biologisch wirksamen hybriden nukleären Rezeptors in einer Zelle fähig ist, wobei das erste DNA-Konstrukt einen ersten Promotor umfasst, der mit einem ersten DNA-Molekül operabel verbunden ist, wobei das erste DNA-Molekül eine Sequenz umfasst, die für einen ersten biologisch wirksamen nukleären Rezeptor als ein Fusionsprotein an eine erste inaktive mutierte Form eines Reporterenzyms kodiert, und
ein zweites DNA-Konstrukt, das zum Steuern der Expression eines biologisch wirksamen Proteinpartners in einer Zelle fähig ist, wobei das zweite DNA-Konstrukt einen zweiten Promotor umfasst, der mit einem zweiten DNA-Molekül operabel verbunden ist, wobei das zweite DNA-Molekül eine Sequenz umfasst, die für einen biologisch wirksamen Proteinpartner als ein Fusionsprotein an eine zweite inaktive mutierte Form des Reporterenzyms kodiert, wobei die ersten und zweiten inaktiven mutierten Formen des Reporterenzyms bei Bildung eines Komplexes zwischen dem ersten nukleären Rezeptor und dem Proteinpartner wechselwirken, um ein aktives Reporterenzym auszubilden.

36. Zelle nach Anspruch 35, wobei der Proteinpartner ein Co-Faktor für den ersten nukleären Rezeptor ist.

37. Zelle nach Anspruch 35, wobei der Proteinpartner ein zweiter nukleärer Rezeptor ist.

38. Zelle nach Anspruch 37, wobei der zweite nukleäre Rezeptor zu dem ersten nukleären Rezeptor identisch ist.

39. Zelle nach Anspruch 37, wobei der zweite nukleäre Rezeptor zu dem ersten nukleären Rezeptor verschieden ist.

40. Festträger mit einer Vielzahl von darauf abgeschiedenen Zellen, wobei die Zellen exprimieren:
einen ersten nukleären Rezeptor als ein Fusionsprotein an eine erste inaktive mutierte Form eines Reporterenzyms und einen Proteinpartner als ein Fusionsprotein an eine zweite inaktive mutierte Form des Reporterenzyms,
wobei die ersten und zweiten inaktiven mutierten Formen des Reporterenzyms bei Ausbilden eines Komplexes zwischen dem ersten nukleären Rezeptor und dem Proteinpartner wechselwirken, um ein wirksames Reporterenzym auszubilden.

41. Festträger nach Anspruch 40, wobei die Zellen ein Enzymsubstrat umfassen, das eine enzymlabile chemische Gruppe umfasst, die bei Spaltung durch das Reporterenzym ein Produkt freisetzt, das durch Kolorimetrie, Fluoreszenz oder Chemilumineszenz messbar ist.

42. Festträger nach Anspruch 40, wobei der Festträger aus einem Material besteht, das ausgewählt ist aus der Gruppe, bestehend aus Glas, Kunststoff, Keramik, Halbleiter, Silizium, Faseroptik, Diamant, biokompatiblen Monomeren und biokompatiblen polymerischen Materialien.

## Revendications

1. Procédé in vitro pour la détection d'interactions de récepteurs nucléaires, le procédé comprenant:
la fourniture d'une cellule qui exprime: un premier récepteur nucléaire sous la forme d'une protéine de fusion à un premier fragment d'une molécule reporter; et un partenaire protéique sous la forme d'une protéine de fusion à un deuxième fragment de la molécule reporter, tandis que les premier et deuxième fragments de la molécule reporter n'ont pas indépendamment de fonction de reporter et tandis que les premier et deuxième fragments de la molécule reporter interagissent pour restaurer la fonction de reporter lors de la formation d'un complexe entre le premier récepteur nucléaire et le partenaire protéique; et
la détermination de la présence et/ou de la quantité de la molécule reporter;
tandis que la présence de la molécule reporter indique la formation d'un complexe entre le récepteur nucléaire et le partenaire protéique.

2. Procédé selon la revendication 1, dans lequel la molécule reporter est choisie dans le groupe consistant en une enzyme monomère, une enzyme multimère, une protéine fluorescente, une protéine luminescente, et une protéine phosphorescente.

3. Procédé selon la revendication 1, dans lequel le premier fragment de la molécule reporter est une première forme mutante inactive d'une enzyme reporter et dans lequel le deuxième fragment de la molécule reporter est une deuxième forme mutante inactive de l'enzyme reporter, et dans lequel les première et deuxième formes inactives de l'enzyme reporter interagissent lors de la formation d'un complexe entre le premier récepteur nucléaire et le partenaire protéique pour former une enzyme reporter active.

4. Procédé selon l'une quelconque des revendications 1 à 3, dans lequel le partenaire protéique est un cofacteur pour le premier récepteur nucléaire.

5. Procédé selon la revendication 4, dans lequel le cofacteur est un corépresseur ou un coactivateur.

6. Procédé selon la revendication 4, dans lequel le cofacteur est une protéine de type sauvage ou une protéine mutante.

7. Procédé selon la revendication 4, dans lequel l'activité de l'enzyme reporter est détectée in vitro dans une cellule vivante.

8. Procédé selon l'une quelconque des revendications 1 à 3, dans lequel le partenaire protéique est un deuxième récepteur nucléaire.

9. Procédé selon la revendication 8, dans lequel le deuxième récepteur nucléaire est le même que le premier récepteur nucléaire.

10. Procédé selon la revendication 8, dans lequel le deuxième récepteur nucléaire est différent du premier récepteur nucléaire.

11. Procédé selon la revendication 3, dans lequel la cellule contient également un élément de réponse aux hormones lié de manière opérationnelle à un gène reporter de telle sorte que la liaison du complexe récepteur nucléaire/partenaire protéique à l'élément de réponse aux hormones a pour résultat l'expression du gène reporter, le procédé comprenant en outre:
la détermination de la présence et/ou de la quantité d'une protéine reporter de substitution codée par le gène reporter;
tandis que la présence et/ou la quantité de la protéine reporter de substitution est une indication des propriétés d'activation de transcription du complexe récepteur nucléaire/partenaire protéique.

12. Procédé selon la revendication 11, dans lequel la protéine reporter de substitution est une enzyme reporter de substitution qui est différente de l'enzyme reporter.

13. Procédé selon la revendication 12, dans lequel l'enzyme reporter de substitution est la luciférase.

14. Procédé selon la revendication 3, comprenant en outre:
l'exposition de la cellule à un composé comprenant un ou plusieurs ligands;
tandis qu'une activité d'enzyme reporter accrue indique une activité d'agoniste du composé et qu'une activité d'enzyme reporter réduite indique une activité d'agoniste inverse ou d'antagoniste du composé.

15. Procédé selon la revendication 14, dans lequel le premier récepteur nucléaire est un récepteur nucléaire orphelin.

16. Procédé selon la revendication 3, comprenant en outre:
la lyse des cellules; et
l'incubation du lysat de cellules avec un substrat, tandis que le substrat émet un signal détectable après clivage par l'enzyme reporter.

17. Procédé selon la revendication 16, dans lequel le substrat est un substrat chimioluminescent ou fluorescent.

18. Procédé selon la revendication 16, dans lequel le substrat est un substrat 1,2-dioxétane chimioluminescent.

19. Procédé selon la revendication 12, comprenant en outre:
la lyse des cellules; et
l'incubation du lysat de cellules avec un premier et un deuxième substrats, tandis que le premier substrat émet un premier signal détectable après clivage par l'enzyme reporter et tandis que le deuxième substrat émet un deuxième signal détectable différent du premier signal détectable après clivage par l'enzyme reporter de substitution.

20. Procédé selon la revendication 19, dans lequel le premier et le deuxième substrats sont choisis indépendamment dans le groupe consistant en des substrats chimioluminescents et fluorescents.

21. Procédé selon l'une quelconque des revendications 1 à 3, dans lequel la cellule est choisie dans le groupe consistant en cellules mammaliennes, cellules de nématode, cellules d'insecte, cellules de levure et cellules de bactérie.

22. Molécule d'ADN comprenant une séquence codant pour un récepteur nucléaire hybride biologiquement actif, tandis que le récepteur nucléaire hybride comprend un récepteur nucléaire sous forme d'une protéine de fusion à une forme mutante inactive d'une enzyme reporter.

23. Molécule d'ADN selon la revendication 22, dans laquelle la forme mutante inactive de l'enzyme reporter est un mutant de β-galactosidase.

24. Construction d'ADN capable de diriger l'expression d'un récepteur nucléaire hybride biologiquement actif dans une cellule, la construction d'ADN comprenant les éléments liés de manière opérationnelle suivants:
un promoteur; et
la molécule d'ADN selon la revendication 22.

25. Construction d'ADN selon la revendication 24, dans laquelle la forme mutante inactive de l'enzyme reporter est un mutant de β-galactosidase.

26. Cellule isolée comprenant la construction d'ADN selon la revendication 24.

27. Cellule isolée comprenant la construction d'ADN selon la revendication 25.

28. Molécule d'ADN comprenant une séquence codant pour un hybride biologiquement actif d'un cofacteur de récepteur nucléaire sous forme d'une protéine de fusion à une forme mutante inactive d'une enzyme reporter.

29. Molécule d'ADN selon la revendication 28, dans laquelle la forme mutante inactive de l'enzyme reporter est un mutant de β-galactosidase.

30. Construction d'ADN capable de diriger l'expression d'un hybride biologiquement actif d'un cofacteur de récepteur nucléaire dans une cellule, comprenant les éléments liés de manière opérationnelle suivants:
un promoteur; et
la molécule d'ADN selon la revendication 28.

31. Construction d'ADN selon la revendication 30, dans laquelle la forme mutante inactive de l'enzyme reporter est un mutant de β-galactosidase.

32. Cellule isolée comprenant la construction d'ADN selon la revendication 30.

33. Cellule isolée comprenant la construction d'ADN selon la revendication 31.

34. Molécule d'ADN selon la revendication 28, dans laquelle le cofacteur de récepteur nucléaire est l'ACTR.

35. Cellule isolée comprenant:
une première construction d'ADN capable de diriger l'expression d'un premier récepteur nucléaire hybride biologiquement actif dans une cellule, la première construction d'ADN comprenant un premier promoteur lié de manière opérationnelle à une première molécule d'ADN, la première molécule d'ADN comprenant une séquence codant pour un premier récepteur nucléaire biologiquement actif sous forme d'une protéine de fusion à une première forme mutante inactive d'une enzyme reporter; et
une deuxième construction d'ADN capable de diriger l'expression d'un partenaire protéique biologiquement actif dans une cellule, la deuxième construction d'ADN comprenant un deuxième promoteur lié de manière opérationnelle à une deuxième molécule d'ADN, la deuxième molécule d'ADN comprenant une séquence codant pour un partenaire protéique biologiquement actif sous forme d'une protéine de fusion à une deuxième forme mutante inactive de l'enzyme reporter;
tandis que les première et deuxième formes mutantes inactives de l'enzyme reporter interagissent lors de la formation d'un complexe entre le premier récepteur nucléaire et le partenaire protéique pour former une enzyme reporter active.

36. Cellule selon la revendication 35, dans laquelle le partenaire protéique est un cofacteur pour le premier récepteur nucléaire.

37. Cellule selon la revendication 35, dans laquelle le partenaire protéique est un deuxième récepteur nucléaire.

38. Cellule selon la revendication 37, dans laquelle le deuxième récepteur nucléaire est le même que le premier récepteur nucléaire.

39. Cellule selon la revendication 37, dans laquelle le deuxième récepteur nucléaire est différent du premier récepteur nucléaire.

40. Support solide ayant, déposées sur lui, une pluralité de cellules, tandis que les cellules expriment:
un premier récepteur nucléaire sous forme d'une protéine de fusion à une première forme mutante inactive d'une enzyme reporter; et
un partenaire protéique sous forme d'une protéine de fusion à une deuxième forme mutante inactive de l'enzyme reporter;
tandis que les première et deuxième formes mutantes inactives de l'enzyme reporter interagissent pour former une enzyme reporter active lors de la formation d'un complexe entre le premier récepteur nucléaire et le partenaire protéique.

41. Support solide selon la revendication 40, dans lequel les cellules comprennent un substrat d'enzyme comprenant un groupe chimique labile aux enzymes qui, lors du clivage par l'enzyme reporter, libère un produit mesurable par colorimétrie, fluorescence ou chimioluminescence.

42. Support solide selon la revendication 40, dans lequel le support solide est constitué d'une matière choisie dans le groupe consistant en verre, plastique, céramique, semi-conducteur, silice, fibre optique, diamant, monomères bio-compatibles et matières polymères bio-compatibles.
